# EUROPÄISCHE PATENTSCHRIFT

(11) **EP 0 748 798 B1**
(45) Veröffentlichungstag und Bekanntmachung des Hinweises auf die Patenterteilung: **09.10.2002**
(21) Anmeldenummer: 96108858.0
(22) Anmeldetag: 03.06.1996
(51) Int. Cl.: C07D 213/61, C07C 233/05, C07C 233/06, C07B 37/08

(54) **N-substituierte cis-N-propenyl-acetamide und Verfahren zu ihrer Herstellung**
N-substituted cis-N-propenyl-acetamides and process for their preparation
Cis-N-propényl-acétamides N-substitués et procédé pour leur préparation

(30) Priorität: 14.06.1995 DE 19521588
(43) Veröffentlichungstag der Anmeldung: 18.12.1996
(73) Patentinhaber: BAYER AG, 51368 Leverkusen (DE)
(72) Erfinder: Lantzsch, Reinhard, Dr., 42115 Wuppertal (DE)

(56) Entgegenhaltungen:
- EP-A- 0 546 418
- EP-A- 0 670 302
- EP-A- 0 701 998
- DE-A- 2 334 632
- JOURNAL OF THE CHEMICAL SOCIETY, PERKIN TRANSACTIONS I, 1984, Seiten 1173-82, XP000602179 O. METH-COHN ET AL.: "A Versatile New Synthesis of Quinolines and Related Fused Pyridines. Part 12. A General Synthesis of 2-Chloropyridines and 2-Pyridones"
- JOURNAL OF THE CHEMICAL SOCIETY, PERKIN TRANSACTIONS II, 1973, Seiten 1954-7, XP000602178 A.J. HUBERT ET AL.: "Catalysed Prototropic Rearrangements. Part II. Metal Carbonyl-catalysed Isomerization of N-Allylamides to Prop-2-enyl Derivatives"
- JOURNAL OF ORGANIC CHEMISTRY, Bd. 45, 1980, Seiten 2139-45, XP000602175 J.K. STILLE ET AL.: "Isomerization of N-Allylamides and -imides to Aliphatic Enamides by Iron, Rhodium, and Ruthenium Complexes"
- JOURNAL OF ORGANIC CHEMISTRY, Bd. 46, 1981, Seiten 2899-2901, XP000602176 K.S. NG ET AL.: "Synthesis of Enamides and Amides by Hydrozirconation-Acylation of Schiff Bases"
- JOURNAL OF THE CHEMICAL SOCIETY, CHEMICAL COMMUNICATIONS, 1994, Seiten 2143-4, XP002014411 T. MURAI ET AL.: "Rhodium(II) Acetate Catalysed Hydrosilylation of Enamides and N-Vinylureas leading to 1-(Trialkylsilyl)- alkylamine Derivatives"
- JOURNAL OF THE CHEMICAL SOCIETY, PERKIN TRANSACTIONS I, 1993, Seiten 2339-44, XP002014412 S. OZAKI ET AL.: "Indirect Electroreductive Cyclisation of N-Allylic and N-Propargylbromo Amides and o-Bromoacryloylanilides using Nickel(II) Complexes as Electron-transfer Catalysts"

## Beschreibung

Die Erfindung betrifft neue N-substituierte cis-N-Propenyl-acetamide, welche als Zwischenprodukte zur Herstellung von agrochemischen Wirkstoffen verwendet werden können, und ein Verfahren zu ihrer Herstellung.

Es ist bereits bekannt, daß man N-Alkenyl-acetamide der Formel (A) erhält, wenn man Imine der Formel (B) mit Acetanhydrid oder Acetylchlorid, gegebenenfalls in Gegenwart eines Säureakzeptors gemäß dem folgenden Reaktionsschema umsetzt: 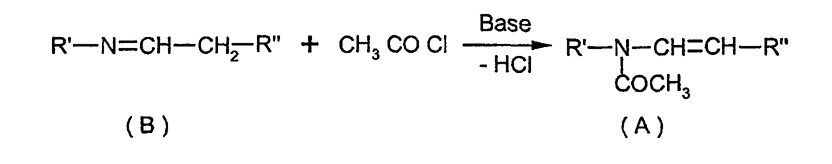 (vgl. J. Chem. Soc. Perkin Trans. I 1984, 1173-82 und EP-A 0 546 418)

Dabei entstehen jedoch die entsprechenden trans-Derivate; die Herstellung von cis-Derivaten ist noch nicht beschrieben.

Gegenstand der vorliegenden Erfindung sind
1) neue N-substituierte cis-N-Propenyl-acetamide der allgemeinen Formel (I) 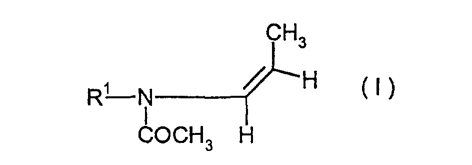 in welcher
   - R¹: für gegebenenfalls durch C₁-C₂-Alkoxy substituiertes C₁-C₆-Alkyl steht;
   für 1-Buten-4-yl, 2-Buten-4-yl; Propinyl oder Butinyl steht;
   für Cyclopropyl, Cyclobutyl, Cyclopentyl, Cyclohexyl, Cyclopropylmethyl, Cyclobutylmethyl, Cyclopentylmethyl oder Cyclohexylmethyl steht oder
   für jeweils gegebenenfalls einfach bis dreifach, gleich oder verschieden substituiertes Benzyl, Phenylethyl, Phenylpropyl, Naphthylmethyl, Thienylmethyl oder Pyridylmethyl steht, wobei als Substituenten jeweils genannt seien: Halogen, C₁-C₄-Alkyl und C₁-C₄-Alkoxy;
2) ein Verfahren zur Herstellung von N-substituierten cis-N-Propenyl-acetamiden der allgemeinen Formel (I) 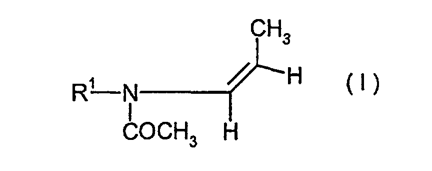 in welcher
   - R¹: die oben angegebene Bedeutung hat,
   dadurch gekennzeichnet, daß man Acetamide der allgemeinen Formel (II) 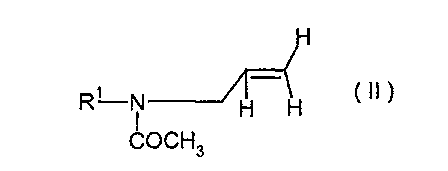 in welcher
   - R¹: die oben angegebene Bedeutung hat,
   mit Alkalimetallhydroxiden oder Alkalimetallalkoholaten, gegebenenfalls in Gegenwart eines aprotischen, polaren Lösungsmittels isomerisiert;
   und
3) die Verwendung der N-substituierten cis-N-Propenyl-acetamide der allgemeinen Formel (I) zur Herstellung des 2-Chlor-5-methyl-pyridins der Formel (IV) 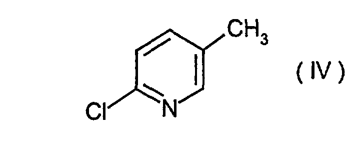 durch Umsetzung mittels "Vilsmeier Reagenz", d.h. mit einem Formamid-Derivat der allgemeinen Formel (III) 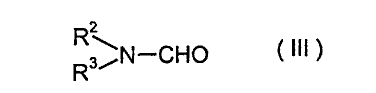
in welcher
- R² und R³: gleich oder verschieden sind und für C₁-C₆-Alkyl oder C₃-C₆-Cycloalkyl stehen oder gemeinsam für C₂-C₆-Alkandiyl stehen,
sowie mit einem Chlorierungsmittel, gegebenenfalls in Gegenwart eines organischen Lösungsmittels bei Temperaturen zwischen -30°C und 150°C.

Überraschenderweise können die neuen N-substituierten cis-N-Propenyl-acetamide der Formel (I) nach dem erfindungsgemäßen Verfahren auf einfache Weise und in sehr guten Ausbeuten erhalten werden, obwohl ähnliche Isomerisierungen bisher nur photochemisch mit Eisencarbonylen als Umlagerungskatalysatoren beschrieben sind (vgl. z.B. J. Chem. Soc. Perkin Trans II, 1973, S. 1954-57). Das erfindungsgemäße Verfahren eignet sich somit vor allem auch für eine Herstellung im technischen Maßstab, was eine wertvolle Bereicherung des Standes der Technik darstellt.

N-substituierte cis-N-Propenyl-acetamide der Formel (I) dienen zur Herstellung von 2-Chlor-5-methyl-pyridin.

Nach dem erfindungsgemäßen Verfahren werden vorzugsweise Verbindungen der Formel (I) hergestellt, in welcher
- R¹: für jeweils gegebenenfalls durch Methoxy oder Ethoxy substituiertes Methyl, Ethyl, n- oder i-Propyl und n-, i-, s- oder t-Butyl steht;
für jeweils gegebenenfalls einfach bis zweifach, gleich oder verschieden substituiertes Benzyl oder Phenethyl steht, wobei als Substituenten genannt seien: Fluor, Chlor, Brom, Methyl, Ethyl, n- oder i-Propyl, n-, i-, s- oder t-Butyl, Methoxy und Ethoxy;
für Cyclohexyl oder für Chlorpyridylmethyl steht.

Die oben aufgeführten allgemeinen oder in Vorzugsbereichen angegebenen Restedefinitionen gelten sowohl für die Endprodukte der Formel (I), als auch entsprechend für die zur Herstellung benötigten Ausgangsstoffe.

Verwendet man beispielsweise N-Methyl-N-allylacetamid als Ausgangsstoff und Kaliumhydroxid als Base, so kann der Reaktionsablauf des erfindungsgemäßen Verfahrens durch das folgende Formelschema wiedergegeben werden:

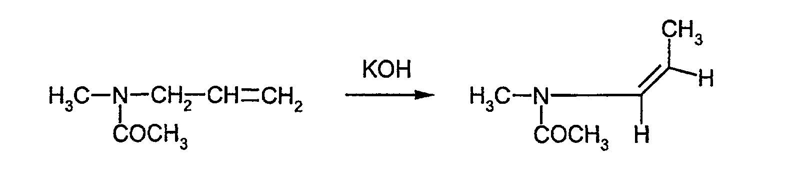

Die beim erfindungsgemäßen Verfahren zur Herstellung der Verbindungen der Formel (I) als Ausgangsstoffe zu verwendenden Acetamide sind durch die Formel (II) allgemein definiert. In der Formel (II) hat R¹ vorzugsweise diejenigen Bedeutungen, die bereits oben im Zusammenhang mit der Beschreibung der neuen Verbindungen der Formel (I) als bevorzugt für R¹ angegeben wurden.

Die Acetamide der Formel (II) sind bekannt und/oder können nach an sich bekannten Verfahren hergestellt werden (vgl. z.B. DE-OS 23 34 632 oder J. Chem. Soc. Perkin Trans. I 1993, 2344).

Die erfindungsgemäße Isomerisierung erfolgt in Gegenwart von Alkalimetallhydroxiden, wie Lithiumhydroxid, Natriumhydroxid oder Kaliumhydroxid, oder von Alkalimetallalkoholaten, wie Natriummethylalt, Natriumethylat, Natriumisopropylat, Natrium-n-butylat, Natrium-sec.-butylat, Natrium-tert.-butylat, Kalium-n-butylat, Kalium-sec.-butylat oder Kalium-tert.-butylat.

Die erfindungsgemäße Isomerisierung wird gegebenenfalls in Gegenwart eines aprotischen, polaren Lösungsmittels durchgeführt. Hierzu gehören insbesondere Ether, wie Methyl-tert.-butylether, Methyl-tert.-amylether, 1,2-Dimethoxoxyethan; Diethoxymethan, Ethylenglykoldimethyl- oder diethylether, ferner Nitrile wie Acetonitril, Propionitril, Benzonitril oder Butyronitril, ferner Amide, wie Formamid, Dimethylformamid, Diethylformamid, Di-Cyclohexylformamid, Dibutylformamid, Acetamid, Dimethylacetamid, N-Methyl-pyrrolidon, N-Methyl-E-caprolactam, Harnstoffe, wie Tetramethylharnstoff, Sulfoxide und Sulfone, wie Dimethylsulfoxid oder Sulfolan.

Die Reaktionstemperaturen können bei der Durchführung der erfindungsgemäßen Isomerisierung in einem größeren Bereich variiert werden. Im allgemeinen arbeitet man bei Temperaturen zwischen -20 und +150°C, vorzugsweise bei Temperaturen zwischen 0°C und 100°C, je nach verwendeter Base und/oder gegebenenfalls verwendetem Lösungsmittel, um eine hinreichende Reaktionsgeschwindigkeit zu erzielen.

Zur Durchführung der erfindungsgemäßen Isomerisierung setzt man pro Mol Acetamid der Formel (II) im allgemeinen zwischen 0,01 und 2 Mol, insbesondere zwischen 0,05 und 1 Mol Base ein.

Die Aufarbeitung kann in üblicher Weise mittels Wasser erfolgen. Nach Neutralisation der Base wird mit einem geeigneten Lösungsmittel extrahiert. Die Verbindungen können durch Destillation gereinigt werden. Verwendet man katalytische Basenmengen und kein Lösungsmittel oder ein für die nächste Stufe geeignetes Lösungsmittel, kann man die Verbindungen auch ohne Isolierung und Reinigung direkt weiter zum 2-Chlor-5-methyl-pyridin der Formel (IV) umsetzen.

Die nach der erfindungsgemäßen Isomerisierung herstellbaren N-substituierten cis-N-Propenyl-acetamide der Formel (I) können durch Reaktion mit einem Formamid-Derivat der Formel (III) sowie mit einem Chlorierungsmittel zum 2-Chlor-5-methylpyridin der Formel (TV) umgesetzt werden.

Das so herstellbare 2-Chlor-5-methyl-pyridin kann als Zwischenprodukt für Insektizide verwendet werden (vgl. z.B. EP-A 0 163 855).

Überraschenderweise kann das 2-Chlor-5-methylpyridin auf diesem Weg in einfacher Weise und in guten Ausbeuten erhalten werden, obwohl die sterische Anordnung der Substituenten bei den N-substituierten cis-N-Propenyl-acetamiden der Formel (I) für einen Pyridin-Ringschluß ungeeignet erscheint:

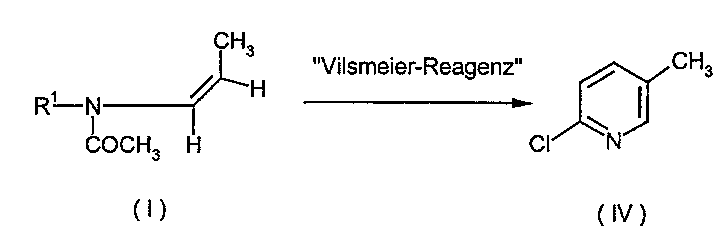

Die außerdem zur Herstellung des 2-Chlor-5-methyl-pyridins der Formel (IV) als Ausgangsstoffe zu verwendenden Formamid-Derivate sind durch die Formel (III) allgemein definiert. In der Formel (III) sind R² und R³ gleich oder verschieden und stehen vorzugsweise für Methyl, Ethyl, n- oder i-Propyl, n-, i-, s- oder t-Butyl; oder für Cyclopentyl oder Cyclohexyl; oder gemeinsam für Butan-1,4-diyl oder Pentan-1,5-diyl.

Als Beispiele für die Formamid-Derivate der Formel (III) seien genannt:
N,N-Dimethyl-formamid, N,N-Diethyl-formamid, N,N-Dipropyl-formamid, N,N-Dibutyl-formamid, N-Cyclohexyl-N-methyl-formamid, N,N-Dicyclohexyl-formamid.

Die Formamid-Derivate der Formel (III) sind bekannte organische Synthesechemikalien.

Das Verfahren zur Herstellung des 2-Chlor-5-methyl-pyridins der Formel (IV) wird unter Verwendung eines Chlorierungsmittels durchgeführt. Es können hierbei die üblichen Chlorierungsmittel verwendet werden, beispielsweise Phosphorylchlorid (Phosphoroxychlorid), Phosphor(V)chlorid, Phosgen, Oxalylchlorid, Thionylchlorid, Perchlorbutansäurechlorid, Dichlorbenzodioxol, N,N-Dimethyl-chlormethylimmoniumchlorid oder N,N-Diethyl-chlormethylimmoniumchlorid.

Das Verfahren zur Herstellung des 2-Chlor-5-methyl-pyridins der Formel (IV) wird gegebenenfalls in Gegenwart eines organischen Lösungsmittels durchgeführt. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Nitrile, wie Acetonitril, Propionitril oder Butyronitril; Amide, wie N,N-Dimethylformamid, N,NDimethylacetamid, N-Methylformanilid, N-Methyl-pyrrolidon oder Hexamethylphosphorsäuretriamid; Sulfoxide wie Dimethylsulfoxid, sowie Sulfone, wie Tetramethylensulfon.

Die Reaktionstemperaturen können bei der Durchführung des Verfahrens zur Herstellung des 2-Chlor-5-methyl-pyridins der Formel (IV) in einem größeren Bereich variiert werden. Im allgemeinen arbeitet man bei Temperaturen zwischen -30°C und +150°C, vorzugsweise bei Temperaturen zwischen -10°C und +120°C, wobei in der Anfangsphase vorzugsweise zwischen -10°C und +40°C und anschließend zwischen +20°C und 120°C gearbeitet wird. Man kann jedoch auch direkt bei erhöhter Temperatur arbeiten und das "Vilsmeier Reagenz" in situ erzeugen.

Das Verfahren wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des Verfahrens zur Herstellung des 2-Chlor-5-methyl-pyridins der Formel (IV) setzt man pro Mol N-substituiertem cis-N-Propenylacetamid der Formel (I) im allgemeinen zwischen 1 und 10 Mol, vorzugsweise zwischen 1,5 bis 5,0 Mol, insbesondere zwischen 2,0 und 4,0 Mol Chlorierungsmittel und zwischen 1 und 10 Mol, vorzugsweise zwischen 2,0 und 4,0 Mol Formamid-Derivat der Formel (III) ein.

Die Aufarbeitung kann auf übliche Weise durchgeführt werden.

### Herstellungsbeispiele

### Beispiel 1

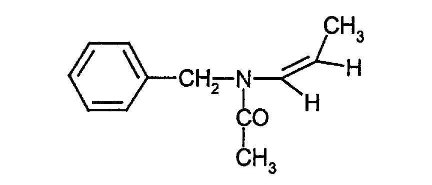

### (Base: Kalium-tert.-Butylat; LM: 1,2-Dimethoxiethan)

9,47 g (0,05 Mol) N-Benzyl-N-allylacetamid werden in 50 ml trockenem 1,2-Dimethoxiethan gelöst. Unter Schutzgas werden bei 20°C 5,6 g (0,05 Mol) Kaliumtert.-Butylat zugegeben. Die Reaktion ist exotherm und die Lösung färbt sich dunkelrot. Man erhitzt eine Stunde unter Rühren auf 40°C, setzt Eiswasser zu und stellt auf pH 7. Nach dreimaligem Extrahieren mit Methylenchlorid werden die vereinigten organischen Phasen getrocknet und eingeengt.

Man erhält 9,3 g eines roten Öls, das durch Destillation im Kugelrohr gereinigt wird (Manteltemperatur 150°C, 0,5 mbar).

Man erhält 8,3 g (87,6 % der Theorie) cis-N-Benzyl-N-propenyl-acetamid vom Brechungsindex = 1,535.
¹H-NMR (CDCl₃, d in ppm): 1.42-1.45 (dd, CH₃), 2.05 (s, CH₃), 4.63 (s, CH₂), 5.47-5,52 (m, CH), 5.97-6.00 (dd, CH), ∼7.3 (m, 5H).

### (Base: Kaliumhydroxid; LM: Dimethylsulfoxid)

9,47 g (0,05 Mol) N-Benzyl-N-allylacetamid werden in 30 ml Dimethylsulfoxid gelöst und 0,28 g (0,005 Mol) Kaliumhydroxid zugegeben. Man rührt 24 Stunden bei Raumtemperatur, destilliert die Hauptmenge des Lösungsmittels im Vakuum ab, versetzt mit Eiswasser und stellt neutral. Die Aufarbeitung erfolgt wie oben.

Man erhält (ohne Destillation) 9,1 g; Gehalt: 91,3 %. Das entspricht einer Ausbeute von 87,7 % der Theorie.

### (Base: Kaliumhydroxid; LM: ohne)

9,47 g (0,05 Mol) N-Benzyl-N-allylacetamid und 0,66 g (0,01 Mol) Kaliumhydroxidpulver (85 %-ig) werden unter Rühren ohne Lösungsmittel eine Stunde auf 80°C erwärmt. Man kühlt ab, gibt Eiswasser zu, stellt mit verdünnter Salzsäure auf pH 8 und extrahiert dreimal mit Methylenchlorid. Die organischen Phasen werden vereinigt, getrocknet und das Lösungsmittel unter vermindertem Druck abdestilliert (Rotationsverdampfer).

Man erhält 9,1 g eines orangenen Öls, das einem Gehalt von N-Benzyl-N-cis-propenylacetamid von 87,5 % (GC) besitzt und bei Bedarf durch Destillation weiter gereinigt werden kann.

Entsprechend Beispiel 1 sowie gemäß den allgemeinen Angaben zur Herstellung können beispielsweise die in der nachfolgenden Tabelle aufgeführten Verbindungen der Formel (I) erhalten werden:

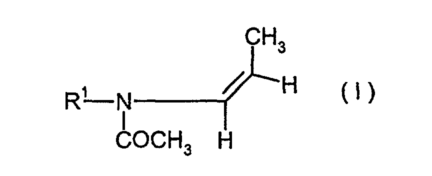

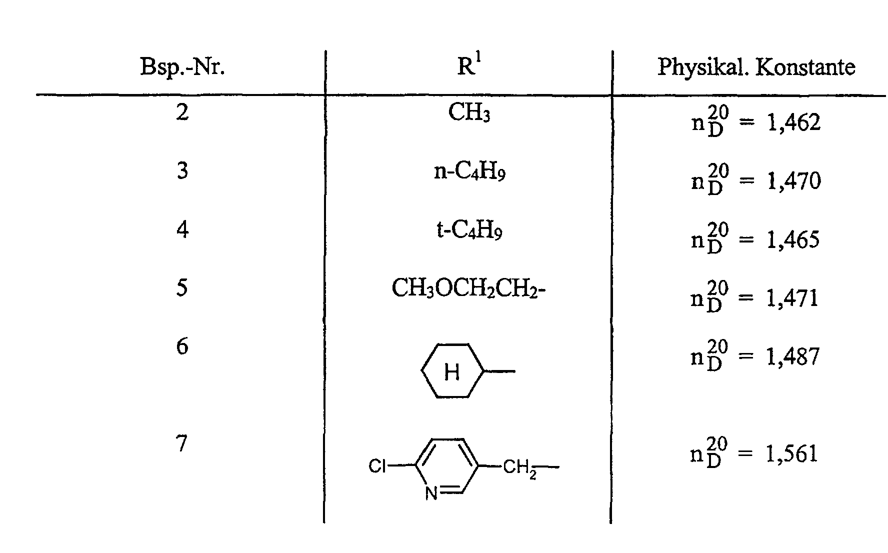

### Herstellung des 2-Chlor-5-methyl-pyridins (IV)

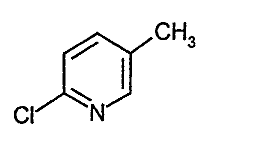

10,4 g (Gehalt: 91,3 %, 0,05 Mol) cis-N-Benzyl-N-propenyl-acetamid werden in 25 ml Acetonitril gelöst. Dann gibt man 11 g (0,15 Mol) Dimethylformamid zu und tropft anschließend bei 20-25°C 19 g (0,15 Mol) Oxalylchlorid unter Kühlung hinzu. Anschließend erhitzt man 18 Stunden unter Rückfluß, kühlt ab und destilliert das Lösungsmittel im Vakuum ab. Das Reaktionsprodukt wird mit Eiswasser verrührt und dreimal mit Methylenchlorid extrahiert. Die organischen Phasen werden vereinigt, getrocknet und eingeengt.

Man erhält 11,8 g einer klaren Flüssigkeit, die zu 47 % aus 2-Chlor-5-methyl-pyridin besteht, das durch Destillation gereinig werden kann. Ausbeute: 86,9 % der Theorie.

## Patentansprüche

1. N-substituierte cis-N-Propenyl-acetamide der allgemeinen Formel (I) 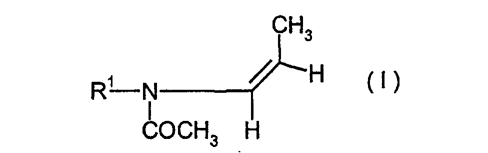 in welcher
R¹ für gegebenenfalls durch C₁-C₂-Alkoxy substituiertes C₁-C₆-Alkyl steht;
für 1-Buten-4-yl, 2-Buten-4-yl; Propinyl oder Butinyl steht;
für Cyclopropyl, Cyclobutyl, Cyclopentyl, Cyclohexyl, Cyclopropylmethyl, Cyclobutylmethyl, Cyclopentylmethyl oder Cyclohexylmethyl steht oder
für jeweils gegebenenfalls einfach bis dreifach, gleich oder verschieden substituiertes Benzyl, Phenylethyl, Phenylpropyl, Naphthylmethyl, Thienylmethyl oder Pyridylmethyl steht, wobei als Substituenten jeweils genannt seien: Halogen, C₁-C₄-Alkyl und C₁-C₄-Alkoxy.

2. Verbindungen der Formel (I) gemäß Anspruch 1, in welcher
R¹ für jeweils gegebenenfalls durch Methoxy oder Ethoxy substituiertes Methyl, Ethyl, n- oder i-Propyl und n-, i-, s- oder t-Butyl steht;
für jeweils gegebenenfalls einfach bis zweifach, gleich oder verschieden substituiertes Benzyl oder Phenethyl steht, wobei als Substituenten genannt seien: Fluor, Chlor, Brom, Methyl, Ethyl, n- oder i-Propyl, n-, i-, s- oder t-Butyl, Methoxy und Ethoxy;
für Cyclohexyl oder für Chlorpyridylmethyl steht.

3. Verfahren zur Herstellung von N-substituierten cis-N-Propenyl-acetamiden der allgemeinen Formel (I) gemäß Anspruch 1 oder 2, **dadurch gekennzeichnet, daß** man Acetamide der allgemeinen Formel (II) 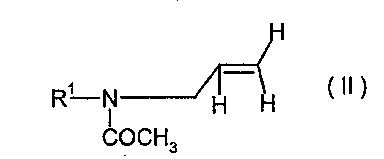 in welcher
R¹ die in Anspruch 1 angegebene Bedeutung hat,
mit Alkalimetallhydroxiden oder Alkalimetallalkoholaten, gegebenenfalls in Gegenwart eines aprotischen, polaren Lösungsmittels isomerisiert.

4. Verwendung der N-substituierten cis-N-Propenyl-acetamide der allgemeinen Formel (I) gemäß Anspruch 1 oder 2 zur Herstellung des 2-Chlor-5-methylpyridins der Formel (IV) 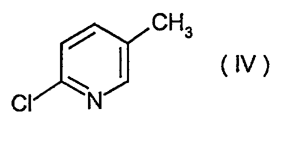 durch Umsetzung mittels "Vilsmeier Reagenz", d.h. mit einem Formamid-Derivat der allgemeinen Formel (III) 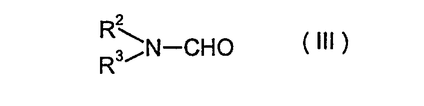 in welcher
R² und R³ gleich oder verschieden sind und für C₁-C₆-Alkyl oder C₃-C₆-Cycloalkyl stehen oder gemeinsam für C₂-C₆-Alkandiyl stehen,
sowie mit einem Chlorierungsmittel, gegebenenfalls in Gegenwart eines organischen Lösungsmittels bei Temperaturen zwischen -30°C und 150°C.

## Claims

1. N-substituted cis-N-propenyl-acetamides of the general formula (I) 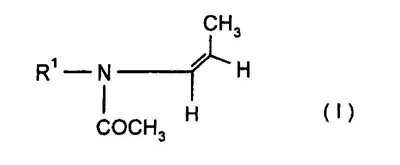 in which
R¹ represents C₁-C₆-alkyl which is optionally substituted by C₁-C₂-alkoxy;
or represents 1-buten-4-yl, 2-buten-4-yl; propinyl or butinyl;
or represents cyclopropyl, cyclobutyl, cyclopentyl, cyclohexyl, cyclopropylmethyl, cyclobutylmethyl, cyclopentylmethyl or cyclohexylmethyl, or represents
benzyl, phenylethyl, phenylpropyl, naphthylmethyl, thienylmethyl or pyridylmethyl, each of which is optionally monosubstituted to trisubstituted by identical or different substituents, substituents which may be mentioned in each case being: halogen, C₁-C₄-alkyl and C₁-C₄-alkoxy.

2. Compounds of the formula (I) according to Claim 1, in which
R¹ represents methyl, ethyl, n- or i-propyl and n-, i-, s- or t-butyl, each of which is optionally substituted by methoxy or ethoxy;
or represents benzyl or phenethyl, each of which is optionally monosubstituted to disubstituted by identical or different substituents, substituents which may be mentioned being: fluorine, chlorine, bromine, methyl, ethyl, n- or i-propyl, n-, i-, s- or t-butyl, methoxy and ethoxy;
or represent cyclohexyl or chloropyridylmethyl.

3. Process for the preparation of N-substituted cis-N-propenyl-acetamides of the general formula (I) according to Claim 1 or 2, **characterized in that** acetamides of the general formula (II) 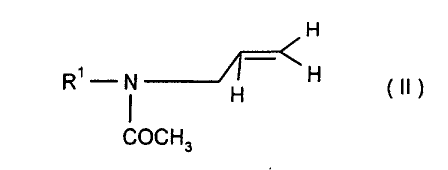 in which
R¹ has the meaning given in Claim 1
are isomerized using alkali metal hydroxides or alkali metal alcoholates, if appropriate in the presence of an aprotic, polar solvent.

4. Use of N-substituted cis-N-propenyl-acetamides of the general formula (I) according to Claim 1 or 2 for the preparation of 2-chloro-5-methyl-pyridine, of the formula (IV) 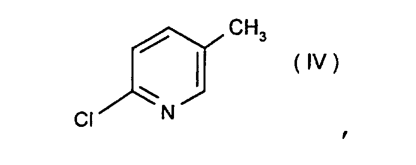 by reaction by means of "Vilsmeier reagent", i.e. with a formamide derivative of the general formula (III) 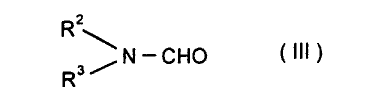 in which
R² and R³ are identical or different and represent C₁-C₆-alkyl or C₃-C₆-cycloalkyl, or together represent C₂-C₆-alkanediyl,
and with a chlorinating agent, at temperatures between -30 and 150°C, if appropriate in the presence of an organic solvent.

## Revendications

1. Cis-N-propénylacétamides N-substitués de la formule générale (I) : 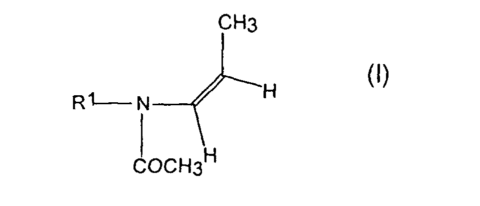 dans laquelle :
R¹ représente un radical alcoyle en C₁-C₆, le cas échéant substitué par un radical alcoxy en C₁-C₂ ;
représente les radicaux 1-butèn-4-yle, 2-butène-4-yle, propynyle ou butynyle ;
représente les radicaux cyclopropyle, cyclobutyle, cyclopentyle, cyclohexyle, cyclopropylméthyle, cyclobutylméthyle, cyclopentylméthyle ou cyclohexylméthyle, ou
représente les radicaux benzyle, phényléthyle, phénylpropyle, naphtylméthyle, thiénylméthyle ou pyridylméthyle, le cas échéant substitués une à trois fois, de manière identique ou différente, où comme substituants on citera :
un atome d'halogène, les radicaux alcoyle en C₁-C₄ et alcoxy en C₁-C₄.

2. Composés de la formule (I) suivant la revendication 1, dans lesquels :
R¹ représente les radicaux méthyle, éthyle, n- ou i-propyle et n-, i-, s- ou t-butyle, le cas échéant substitués par le radical méthoxy ou éthoxy ;
représente le radical benzyle ou phénéthyle, chaque fois le cas échéant substitué une à deux fois, de manière identique ou différente, où comme substituant, on citera les atomes de fluor, de chlore, de brome, les radicaux méthyle, éthyle, n- ou i-propyle et n-, i-, s- ou t-butyle, méthoxy et éthoxy ;
représente le radical cyclohexyle ou chloropyridylméthyle.

3. Procédé de préparation des cis-N-propénylacétamides N-substitués de la formule générale (I) suivant la revendication 1 ou 2, **caractérisé en ce que** l'on isomérise les acétamides de la formule générale (II) : 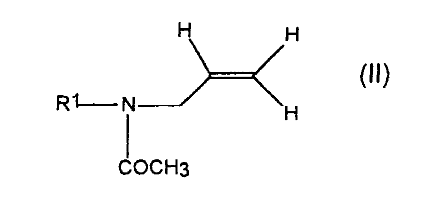 dans laquelle :
R¹ a la signification indiquée à la revendication 1,
avec des hydroxydes de métal alcalin ou des alcoolates de métal alcalin, le cas échéant en présence d'un solvant polaire, aprotique.

4. Utilisation des cis-N-propénylacétamides N-substitués de la formule générale (I) suivant la revendication 1 ou 2, pour la préparation de la 2-chloro-5-méthylpyridine de la formule (IV) ; 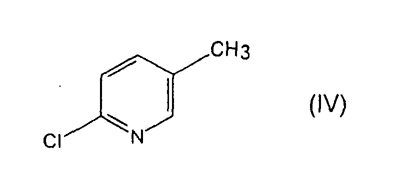 par réaction avec le « réactif de Vilsmeier », à savoir un dérivé formamide de la formule générale (III) : 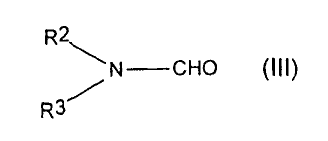 dans laquelle :
R² et R³ sont identiques ou différents et représentent un radical alcoyle en C₁-C₆ ou cycloalcoyle en C₃-C₆ ou représentent ensemble un radical alcanediyle en C₂-C₆,
ainsi qu'avec un agent de chloration, le cas échéant en présence d'un solvant organique, à des températures allant de -30°C à 150°C.
